# EUROPEAN PATENT SPECIFICATION

(11) **EP 1 171 114 B1**
(45) Date of publication and mention of the grant of the patent: **12.11.2008**
(21) Application number: 00905786.0
(22) Date of filing: 27.01.2000
(51) Int. Cl.: A61K 31/197, A61P 13/10

(54) **METHOD FOR THE TREATMENT OF INCONTINENCE**
VERFAHREN ZUR BEHANDLUNG VON INKONTINENZ
METHODE DE TRAITEMENT POUR L'INCONTINENCE

(30) Priority: 08.04.1999 US 128347 P
(43) Date of publication of application: 16.01.2002
(60) Divisional application: 08160836.6
(73) Proprietor: Warner-Lambert Company LLC, New York, NY 10017 (US)
(72) Inventor: SEGAL, Catherine, A., Chester, NJ 07930 (US); MAGNUS, Leslie, Livingston, NJ 07039 (US)
(74) Representative: Ruddock, Keith Stephen
(86) International application number: PCT/US2000/002141
(87) International publication number: WO 2000/061135

(56) References cited:
- WO-A-00/12692
- WO-A-92/09560
- WO-A-98/09948
- GIL-NAGEL A ET AL: "Incontinence during treatment with gabapentin" NEUROLOGY, MAY 1997, 48 (5) P1467-8, XP000910004 UNITED STATES
- DOHERTY ET AL: "Gabapentin in a medically refractory epilepsy population: Seizure response and unusual side effects." EPILEPSIA, , vol. 36, no. 4, 1995, page 71 XP002139721
- IGAWA Y ET AL: "Effects of GABA-receptor stimulation and blockade on micturition in normal rats and rats with bladder outflow obstruction." JOURNAL OF UROLOGY, AUG 1993, 150 (2 PT 1) P537-42, XP000909994 UNITED STATES
- MAGGI CA ET AL: "Neuroeffector mechanisms in the voiding cycle of the guinea-pig urinary bladder." JOURNAL OF AUTONOMIC PHARMACOLOGY, DEC 1987, 7 (4) P295-308, XP000909967 ENGLAND

## Description

### BACKGROUND OF THE INVENTION

### 1. Field Of The Invention

The present disclosure relates to the use of analogs of gamma-aminobutyric acid (GABA) for the treatment of incontinence.

### 2. Description of Related Art

GABA analogs are known agents useful in antiseizure therapy for central nervous system disorders such as epilepsy, Huntington's chorea, cerebral ischemia, Parkinson's disease, tardive dyskinesia, and spasticity. It has also been suggested that the compounds can be used as antidepressants, anxiolytics, and antipsychotics. See WO 92/09560 (United States Serial Number 618,692 filed November 27, 1990) and WO 93/23383 (United States Serial Number 886,080 filed May 20, 1992).

WO 97/33858 teaches that compounds related to gabapentin are useful for treating epilespy, faintness attacks, hypokinesia, cranial disorders, neurodegenerative disorders, depression, anxiety, panic, pain, and neuropathological disorders. WO 97/33858 does not specify what forms of pain are treated.

Additionally, the compounds of the disclosure are known for treatment of neuropathic pain. For example, see Rosner H; Rubin L; Kestenbaum A., Gabapentin adjunctive therapy in neuropathic pain states. Clin J Pain, 1996 Mar, 12:1, 56-8; Segal AZ; Rordorf G., Gabapentin as a novel treatment for postherpetic neuralgia. Neurology, 1996 Apr, 46:4, 1175-6; Wetzel CH; Connelly JF., Use of gabapentin in pain management. Ann Pharmacother, 1997 Sep, 31:9, 1082-3; Zapp JJ., Postpoliomyelitis pain treated with gabapentin [letter]. Am Fam Physician, 1996 Jun, 53:8, 2442, 2445; Cheville A, et al., Neuropathic pain in radiation myelopathy:a case report. Program book, American Pain Society (14th Annual Scientific Meeting). Abstract #95823, p. A-115; Sist T; Filadora V; Miner M; Lema M., Gabapentin for idiopathic trigeminal neuralgia: report of two cases. Neurology, 1997 May, 48:5, 1467; Waldman SD, Tutorial 28: Evaluation and Treatment of Trigeminal Neuralgia. Pain Digest (1997) 7:21-24; Mellick LB; Mellick GA., Successful treatment of reflex sympathetic dystrophy with gabapentin [letter]. Am J Emerg Med, 1995 Jan, 13:1, 96; Mellick GA; Seng MI., The use of gabapentin in the treatment of reflex sympathetic dystrophy and a phobic disorder. Am J Pain Manage 1995; 5:7-9; Mellick GA; Mellicy LB; Mellick LB., Gabapentin in the management of reflex sympathetic dystrophy [letter]. J Pain Symptom Manage, 1995 May, 10:4, 265-6; Mellick GA; Mellick LB., Reflex sympathetic dystrophy treated with gabapentin. Arch Phys Med Rehabil, 1997 Jan, 78:1, 98-105 and Mackin GA., Medical and pharmacologic management of upper extremity neuropathic pain syndromes. J Hand Ther, 1997 Apr-Jun, 10:2, 96-109.

Urinary incontinence (UI) is often described as either urge incontinence, where urine lost is associated with a sudden or strong desire to void, or stress incontinence, where urine loss is associated with coughing, laughing, or physical exercise. A more general category, mixed incontinence, includes those patients showing both stress and urge symptoms.

Although urinary incontinence is quite prevalent, it is still under-diagnosed and under-reported. The U.S. Department of Health and Human Services estimates that UI affects over 13 million Americans at a cost in excess of #15 billion per year. Many victims of UI do not seek help because of embarrassment or a perception that nothing can be done about their problem. Consequently, the general health and social life of these victims may be significantly compromised for years.

The effects on micturition of the GABA receptor agonists muscimol and baclofen are known (see Igawa Y; Mattiasson A; Andersson KE., Effects of GABA-receptor stimulation and blockade on micturition in normal rats and rats with bladder outflow obstruction. Journal of Urology, 1993, 150, 537-542). Furthermore, incontinence has been reported as a side effect during treatment with gabapentin (see Gil-Nagel A, et al., Incontinence during treatment with gabapentin, Neurology, 1997, 48:5, 1467-8; Doherty KP, et al., Gabapentin in a medically refractory epilepsy population: seizure response and unusual side effects, Epilepsia, 1995, 36, Suppl. 4, 71).

### SUMMARY OF THE INVENTION

This invention provides the use of a GABA analog of Formula I 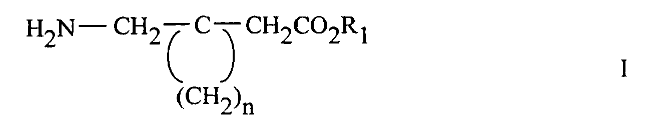 wherein R₁ is hydrogen and n is an integer of from 4 to 6, and the pharmaceutically acceptable salts thereof, for the manufacturing of pharmaceuticals for the treatment of urinary incontinence. An especially preferred embodiment utilizes a compound of Formula I where R₁ is hydrogen and n is 4, which compound is 1-(aminomethyl)-cyclohexane acetic acid, known generically as gabapentin.

### DETAILED DESCRIPTION OF THE PREFERRED EMBODIMENTS

The GABA analogs to be utilized in the method of this disclosure are cyclic amino acids of Formula I. These are described in U.S. Patent 4,024,175.

All that is required to practice the method of this disclosure is to administer a GABA analog of formula I in an amount that is effective to treat incontinence. Such amounts will generally be from about 1 to about 300 mg per kg of subject body weight. Typical doses will be from about 10 to about 5000 mg per day for an adult subject of normal weight. It is expected that common doses that might be administered could be from 100 mg three times a day up to 600 mg four times a day. Commercially available capsules of 100 mg, 300 mg, and 400 mg of gabapentin can be administered. Alternate forms include liquids and film-coated tablets.

While not wishing to be bound by any theory, the inventors believe that the gaba analogs work to control incontinence in the following manner. Incontinence is not associated with pain. A person can sense a full bladder. In overflow incontinence, such as which occurs after a stroke, the feedback loop from the bladder to the brain is broken and the bladder fills and fills until it overflows. This mechanism would be different for urge and stress incontinence. Applicants believe that over sensitivity and irritability of the nerve endings on the bladder sphincter escalate to the point of urge incontinence. Therefore a product that stabilizes and reduces the sensitivity of these nerve fibers breaks the cycle that leads to failure of the muscular control of the sphincter.

The compounds used in the present invention may form pharmaceutically acceptable salts with both organic and inorganic acids or bases. For example, the acid addition salts of the basic compounds are prepared either by dissolving the free base in aqueous or aqueous alcohol solution or other suitable solvents containing the appropriate acid and isolating the salt by evaporating the solution. Examples of pharmaceutically acceptable salts are hydrochlorides, hydrobromides, hydrosulfates, etc. as well as sodium, potassium, and magnesium, etc. salts.

Pharmaceutical compositions of the compound of the present invention or its salts are produced by formulating the active compound in dosage unit form with a pharmaceutical carrier. Some examples of dosage unit forms are tablets, capsules, pills, powders, aqueous and nonaqueous oral solutions and suspensions, and parenteral solutions packaged in containers containing either one or some larger number of dosage units and capable of being subdivided into individual doses.

Some examples of suitable pharmaceutical carriers, including pharmaceutical diluents, are gelatin capsules; sugars such as lactose and sucrose; starches such as corn starch and potato starch, cellulose derivatives such as sodium carboxymethyl cellulose, ethyl cellulose, methyl cellulose, and cellulose acetate phthalate; gelatin; talc; stearic acid; magnesium stearate; vegetable oils such as peanut oil, cottonseed oil, sesame oil, olive oil, corn oil, and oil of theobroma; propylene glycol, glycerin; sorbitol; polyethylene glycol; water; agar; alginic acid; isotonic saline, and phosphate buffer solutions; as well as other compatible substances normally used in pharmaceutical formulations. The compositions of the invention can also contain other components such as coloring agents, flavoring agents, and/or preservatives. These materials, if present, are usually used in relatively small amounts. The compositions can, if desired, also contain other therapeutic agents.

The percentage of the active ingredients in the foregoing compositions can be varied within wide limits, but for practical purposes it is preferably present in a concentration of at least 10% in a solid composition and at least 2% in a primary liquid composition. The most satisfactory compositions are those in which a much higher proportion of the active ingredient is present.

Routes of administration of the subject compound or its salts are oral or parenteral. For example, a useful intravenous dose is between 5 and 50 mg and a useful oral dosage is between 20 and 800 mg. The dosage is within the dosing range used in treatment of pain or as would be with the needs of the patient as described by the physician.

## Claims

1. Use of a compound according to formula I: 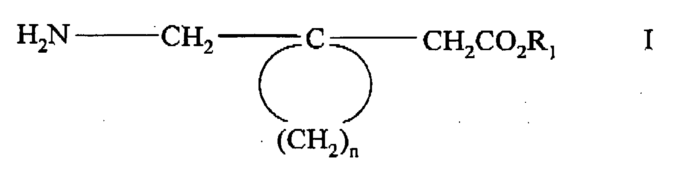 wherein R₁ is hydrogen and n is an integer of from 4 to 6, or a pharmaceutically acceptable salt thereof, for the manufacturing of pharmaceuticals for the treatment of urinary incontinence.

2. The use according to Claim 1, wherein the compound of formula I is gabapentin.

3. The use according to Claim 1, comprising from 10 mg to 400 mg of a compound of the formula 1.

4. The use according to Claim 2, comprising from 10 mg to 400 mg of gabapentin.

## Patentansprüche

1. Verwendung einer Verbindung gemäß Formel I: 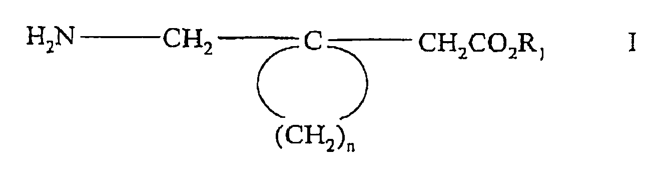 worin R₁ Wasserstoff ist und n eine ganze Zahl von 4 bis 6 ist, oder eines pharmazeutisch verträglichen Salzes davon für die Herstellung von Pharmazeutika für die Behandlung von Harninkontinenz.

2. Verwendung gemäß Anspruch 1, wobei die Verbindung der Formel I Gabapentin ist.

3. Verwendung gemäß Anspruch 1, umfassend von 10 mg bis 400 mg einer Verbindung der Formel I.

4. Verwendung gemäß Anspruch 2, umfassend von 10 mg bis 400 mg Gabapentin.

## Revendications

1. Utilisation d'un composé de formule I : 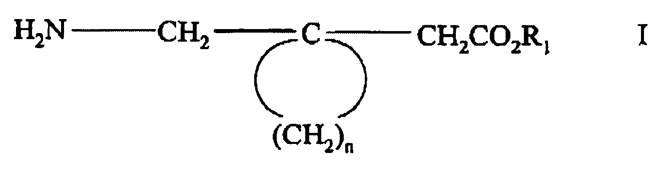 dans laquelle R₁ représente l'hydrogène et n est un entier compris entre 4 et 6, ou d'un sel pharmaceutiquement acceptable correspondant, pour la fabrication d'agents pharmaceutiques pour le traitement de l'incontinence urinaire.

2. Utilisation selon la revendication 1, dans laquelle le composé de formule I est la gabapentine.

3. Utilisation selon la revendication 1, comprenant de 10 mg à 400 mg d'un composé de formule I.

4. Utilisation selon la revendication 2, comprenant de 10 mg à 400 mg de gabapentine.
